# EUROPEAN PATENT APPLICATION

(11) **EP 4 282 480 A1**
(43) Date of publication of application: **29.11.2023**
(21) Application number: 22742626.9
(22) Date of filing: 20.01.2022
(51) Int. Cl.: A61P 25/28, A61K 38/16, A61K 35/748, A61K 31/4025, A23L 33/135

(54) **COMPOSITION FOR SUPPRESSING DETERIORATION OF OR ENHANCING MEMORY LEARNING FUNCTION AND/OR COGNITIVE FUNCTION**

(30) Priority: 25.01.2021 JP 2021009348
(71) Applicant: DIC Corporation, Tokyo 174-8520 (JP)
(72) Inventor: IMAI, Yasuyuki, Ichihara-shi, Chiba 290-8585 (JP); KOSEKI, Yurino, Ichihara-shi, Chiba 290-8585 (JP)
(74) Representative: Gerauer, Marc Philippé
(86) International application number: PCT/JP2022/001882
(87) International publication number: WO 2022/158504

(57) **Abstract**

An object of the invention is to provide a composition for improving memory learning function and/or cognitive function or suppressing deterioration thereof which can be orally taken daily and continuously. As a result of intensive studies, the present inventors have found that the object can be achieved with a composition for improving memory learning function and/or cognitive function or suppressing deterioration thereof containing at least one kind selected from the group consisting of spirulina, phycocyanin, enzymatically degraded spirulina and enzymatically degraded phycocyanin as an active ingredient.

## Description

### TECHNICAL FIELD

The present invention relates to a composition for improving memory learning function and/or cognitive function or suppressing deterioration thereof.

### BACKGROUND ART

Recently, in super-aging society, there is an increasing interest in improving memory learning function and/or cognitive function or suppressing deterioration thereof. It is known that memory learning ability and/or cognitive function deteriorate in elderly people due to aging of the brain and the like. When memory learning ability and/or cognitive function deteriorate, symptoms such as memory impairment including forgetfulness, deterioration of language ability, deterioration of attention/concentration and deterioration of learning ability are caused, which cause troubles in the daily life. Accordingly, development of a food and/or a pharmaceutical product for improving memory learning function and/or cognitive function or suppressing deterioration thereof which can be orally taken daily and continuously is desired.

As foods for improving memory learning function and/or cognitive function or suppressing deterioration thereof, for example, foods containing vitamin C, EPA, DHA, rice bran and the like are currently used. There are, however, still only a small number of foods which exhibit a sufficient effect of improving memory learning function and/or cognitive function or suppressing deterioration thereof. Moreover, although many pharmaceutical products have been studied as pharmaceutical products for treating Alzheimer's disease, which is a cognitive dysfunction, most of the existing agents for treating Alzheimer's disease are mainly based on nerve stimulation action and are merely for symptomatic therapy, and thus the effects thereof are considered to be limited.

Here, with the recent increasing trend toward health, spirulina draws attention as a food which has an excellent nutritional balance and which has a higher nutritional value than general foods. Spirulina refers to cyanobacterium species which are abundant in proteins, saccharides, vitamins, minerals and plant pigments. In addition to the applications of spirulina as foods and supplements for taking many kinds of nutrient with a good balance, it is expected that spirulina itself and a spirulina-derived substance have many functions, and thus studies on application methods of spirulina have been conducted actively.

For example, a lipase activity inhibitor containing phycocyanin as an active ingredient has been reported because spirulina-derived phycocyanin has an action of inhibiting the activity of a lipase such as pancreatic lipase (see PTL 1).

Moreover, a serum lipid-reducing agent containing phycocyanin as an active ingredient has been reported because spirulina-derived phycocyanin has a higher action of improving serum lipid than soybean protein (see PTL 2).

Furthermore, it has been reported that oral administration of spirulina (a spirulina extract) induces activation of natural killer (NK) cells (see NPL 1).

### CITATION LIST

### PATENT LITERATURE

PTL 1: JP2004-359638A
PTL 2: JP2003-137805A

### NON PATENT LITERATURE

NPL 1: Hirahashi T, et al. "Activation of the human innate immune system by spirulina: Augmentation of interferon gamma production and NK cytotoxicity by oral administration of spirulina." International Immunopharmacology 2(2002)423-434.

### SUMMARY OF INVENTION

### TECHNICAL PROBLEM

However, it has not been known so far that spirulina itself or a spirulina-derived substance exhibits an action of improving memory learning function and/or cognitive function or an action of suppressing deterioration of memory learning function and/or cognitive function.

A purpose of the invention is to provide a composition for improving memory learning function and/or cognitive function or suppressing deterioration thereof which can be orally taken daily and continuously.

### SOLUTION TO PROBLEM

As a result of intensive studies to solve the problem, the present inventors have found that spirulina itself and a spirulina-derived substance exhibit an action of improving memory learning function and/or cognitive function and an action of suppressing deterioration of memory learning function and/or cognitive function and thus have completed the invention.

That is, the invention includes the following aspects.
[1] A composition for improving memory learning function and/or cognitive function or suppressing deterioration thereof containing at least one kind selected from the group consisting of spirulina, phycocyanin, enzymatically degraded spirulina and enzymatically degraded phycocyanin as an active ingredient.
[2] The composition described in [1], in which the enzyme in the enzymatically degraded spirulina and the enzymatically degraded phycocyanin is a protease.
[3] The composition described in [1] or [2], in which the phycocyanin is phycocyanin derived from a cyanobacterium.
[4] The composition described in [3], in which the cyanobacterium is a cyanobacterium of the genus *Spirulina.*
[5] The composition described in any of [1] to [4], in which the active ingredient is the phycocyanin or the enzymatically degraded phycocyanin.
[6] The composition described in any of [1] to [5] which is a food composition.
[7] The composition described in [6] which is a health food, a functional food, a nutritional supplement, a supplement, a food with a health claim, a food for a specified health use, a food with a nutrient function claim, a food with a function claim or a food for the ill.
[8] The composition described in any of [1] to [5] which is a pharmaceutical composition.
[9] The composition described in [8] which is a pharmaceutical composition for dementia or Alzheimer's disease.

According to the invention, a composition for improving memory learning function and/or cognitive function or suppressing deterioration thereof which can be orally taken daily and continuously can be provided.

### BRIEF DESCRIPTION OF DRAWINGS

[Fig. 1] A figure showing the change rates of spontaneous alternation behavior in the spontaneous alternation behavior test in Test 1.
[Fig. 2] A figure showing the proportions of the gene expression levels of genes in which the facilitation of gene expression by amyloid β₂₅₋₃₅ was relieved based on the Sham group, in Test 1.
[Fig. 3] A figure showing the proportions of the gene expression levels of genes in which the suppression of gene expression by amyloid β₂₅₋₃₅ was relieved based on the Sham group, in Test 1.

### DESCRIPTION OF EMBODIMENTS

Although the composition according to the embodiment will be explained in detail below, the explanations of components described below are examples as embodiments of the invention, and the invention is not specified by the contents.

The composition according to the embodiment relates to a composition for improving memory learning function and/or cognitive function or suppressing deterioration thereof containing at least one kind selected from the group consisting of spirulina, phycocyanin, enzymatically degraded spirulina and enzymatically degraded phycocyanin as an active ingredient.

Here, examples of the improvement of memory learning function include temporal improvement of memory learning function, promotion of medium- to long-term memory retention, and promotion of development of the brain. Examples of the suppression of deterioration of memory learning function include prevention or suppression of deterioration of memory or learning ability caused with aging.

Moreover, the improvement of cognitive function includes increasing intellectual function such as memory, learning, understanding, judgement and logic, namely cognitive ability, compared to the current state. Examples of the suppression of deterioration of cognitive function include prevention or suppression of deterioration of cognitive ability caused with aging.

After explaining the active ingredient of the composition according to the embodiment below, the application, the form and the like of the composition will be explained.

### <Spirulina>

Spirulina is one active ingredient of the composition according to the embodiment and has an action of improving memory learning function and/or cognitive function and an action of suppressing deterioration of memory learning function and/or cognitive function.

Spirulina is a fine spiral alga belonging to the genus *Spirulina* of the family *Oscillatoriales* of the order Nostocales of the class Cyanophyceae and is abundant in proteins, saccharides, various vitamins, minerals and plant pigments.

Examples of the spirulina include *Spirulina platensis, Spirulina maxima, Spirulina geitleri, Spirulina siamese, Spirulina major, Spirulina subsalsa* (*Spirulina subsalasa*)*, Spirulina princeps, Spirulina laxissima, Spirulina curta,* and *Spirulina spirulinoides.* Particularly preferable spirulina which can be artificially cultured and is thus easily obtained of these is *Spirulina platensis, Spirulina geitleri, Spirulina siamese* or the like. Here, *Spirulina* is also called *Arthrospira.*

As the spirulina used for the composition according to the embodiment, an alga which has been cultured in a liquid medium (wet alga) may be used directly, but a spirulina extract, which is a liquid extract obtained by extracting spirulina in a wet alga with a solvent such as water and ethanol or an extract obtained by concentrating or drying the liquid extract, is preferably used.

### <<Spirulina Extract>>

The extracting liquid used for producing a spirulina extract is not particularly limited in the range in which the effects of the invention are obtained, but, for example, hot water can be used. In the embodiment, a liquid extract obtained by extracting spirulina in an alga with hot water or an extract obtained by concentrating or drying the liquid extract can be preferably used.

The method for obtaining the spirulina extract is not particularly restricted, and the spirulina extract can be obtained according to a general method. Examples thereof include the production methods of a liquid extract described in NPL 1, JPH08-9940A and the like. A specific example is the production method described below.

### <<Production Method of Spirulina Extract>>

The spirulina extract can be produced by extracting from a spirulina alga with hot water at a temperature exceeding 100°C, adjusting the pH of the liquid extract to a specific acidic condition, then removing the insoluble fraction and thus obtaining a spirulina liquid extract.

When the spirulina extract is used in a liquid state, the spirulina liquid extract obtained in the above manner can be used directly. Moreover, the spirulina liquid extract can also be used as powder after concentration or drying. The spirulina extract used for the composition according to the embodiment may be a spirulina liquid extract or an extract produced by concentrating or drying a spirulina liquid extract.

As the spirulina for producing the spirulina extract, commercially available spirulina may be used, or self-cultured spirulina may also be used. Moreover, raw spirulina may be used, or one obtained by drying raw spirulina may also be used.

The culture method for culturing spirulina can be conducted according to a general method which is used for culturing cyanobacteria. For example, spirulina can be cultured and proliferated in the open air under basic conditions.

The spirulina obtained by culturing (also called a spirulina alga below) can be used directly, or cultured spirulina may be collected with filter cloth or filter paper, washed with water and then suspended in water to obtain a suspension. Furthermore, the spirulina may also be a wet alga obtained by concentrating a culture solution or a suspension, a dried alga obtained by drying the wet alga by freeze-drying, spray drying or the like or powder of the dried alga.

The spirulina alga used for the hot-water extraction may be any of a wet alga, a freeze-dried alga, a spray-dried alga, a crushed alga and the like. To obtain a crushed alga, for example, crushing treatment of an alga by a general method, for example, a high-pressure pressing method or the like using a French press in an industrial case, may be used.

Next, the hot-water extraction operation is explained. For example, a spirulina alga processed in the above manner is suspended in advance in an extraction solvent such as distilled water in a pressurized container. The suspension concentration is not particularly restricted but is preferably 1 to 20 mass% based on the solvent considering the extraction efficiency, the costs for recovery and the like. The extraction solvent may be tap water but is preferably distilled water considering that the liquid extract is applied as a food material. The extraction temperature is generally a temperature exceeding 100°C, preferably 105°C to 140°C, more preferably 110 to 130°C. The pressure during the extraction is preferably 1.0 to 2.5 atmospheric pressure. Moreover, a stirring operation may be conducted but does not have to be conducted during the extraction, but a stirring operation is preferably conducted for the thermal efficiency. The extract amount increases as the extraction period becomes longer, but the extraction period is generally preferably 0.5 to 4 hours considering the efficiency.

Next, the algal residue and the protein aggregated through thermal denaturation are removed from the algal residue suspension (pH = around 6.8 to 7.0) after the extraction operation. As the removal operation, for example, an operation such as centrifugation and filtration of the suspension may be conducted, and a supernatant is obtained by the operation. In this regard, however, the supernatant still contains a large amount of dissolved protein, and thus the dissolved protein is preferably further removed. To further remove the dissolved protein, the pH of the liquid extract is preferably adjusted to an acidic condition of the isoelectric point of the protein or lower by adding an acid to the suspension. As a result, the protein can be aggregated, and the aggregated protein can be separated by centrifugation, filtration or the like, thereby obtaining a spirulina extract. Alternatively, without removing the algal residue and the like in the beginning, the algal residue and the aggregated protein may be separated similarly by centrifugation, filtration or the like after adjusting the pH of the algal residue suspension after the extraction operation to the isoelectric point of the protein or lower in the above manner. By removing the aggregated protein, a spirulina extract containing polysaccharides at a high yield can be obtained.

The acidic condition of the isoelectric point of the protein or lower is preferably pH 4.5 or less, more preferably pH 3.75 to 4.25, because the aggregation of the protein and the precipitate formation are the greatest, further preferably pH 4.0.

The acid added for the pH adjustment may be sulfuric acid or hydrochloric acid, but considering the practical operation step and the use as a food material, an organic acid such as citric acid and malic acid is preferably used rather than an inorganic acid.

### <Phycocyanin>

Phycocyanin is a kind of active ingredient of the composition according to the embodiment and has an action of improving memory learning function and/or cognitive function and an action of suppressing deterioration of memory learning function and/or cognitive function.

Phycocyanin is a pigment protein and has phycocyanobilin as a chromophore. Phycocyanin has a structure in which phycocyanobilin and a protein are bound.

Examples of the phycocyanin used for the composition according to the embodiment include phycocyanin derived from an alga such as phycocyanin derived from a cyanobacterium, phycocyanin derived from a red alga and phycocyanin derived from a cryptomonad and the like. Of these, phycocyanin derived from a cyanobacterium is preferable because a large amount can be collected.

Examples of the cyanobacterium include cyanobacteria of the genus *Spirulina,* the genus *Arthrospira,* the genus *Aphanizomenon,* the genus *Fisherella,* the genus *Anabaena,* the genus *Nostoc,* the genus *Synechocystis,* the genus *Synechococcus,* the genus *Tolypothrix,* the genus *Aphanothece,* the genus *Mastigocladis* (*Mastigoclaus*)*,* the genus *Pleurocapsa* and the like. Of these, cyanobacteria of the genus *Spirulina* and the genus *Arthrospira,* which are produced on an industrial scale and which have been found to be safe, are preferable, and a cyanobacterium of the genus *Spirulina* is more preferable.

Moreover, as the raw material for preparing phycocyanin, a raw cyanobacterium may be used, or a dried cyanobacterium may also be used. The dried product of a cyanobacterium may be a dried product obtained from a raw cyanobacterium according to a general method, or a commercial dried product may also be used.

Examples of the phycocyanin include C-phycocyanin, R-phycocyanin, allophycocyanin and the like. In view of the quality, the safety, the easiness of acquisition or the like, C-phycocyanin is preferably contained as the phycocyanin. Accordingly, a preferable embodiment of the composition according to the embodiment is a composition containing C-phycocyanin as an active ingredient. Furthermore, a preferable embodiment of the composition according to the embodiment is a composition containing C-phycocyanin and allophycocyanin. For example, a phycocyanin mixture containing C-phycocyanin and allophycocyanin obtained through extraction from a cyanobacterium of the genus *Spirulina* can be contained in the composition.

Phycocyanin can be obtained, for example, by suspending a cyanobacterium in water or a buffer such as a phosphate buffer and a citrate buffer and extracting the phycocyanin in the cyanobacterium.

The method for extracting phycocyanin is not particularly restricted, and phycocyanin can be extracted according to a general method.

A preferable embodiment of the extraction method is, for example, the extraction method described in JP2006-230272A or the like. A specific example is the extraction method described in the extraction method (i) below. By the extraction method (i) below, high-purity, vivid-colored phycocyanin can be obtained.

### <<Extraction Method (i) of Phycocyanin>>

The extraction method (i) has
a first step of obtaining a liquid extract in which phycocyanin in a cyanobacterium is extracted in an aqueous suspension,
a second step of producing calcium phosphate by reacting a calcium salt and a phosphate in the liquid extract and obtaining an adsorbate by adsorbing a contaminant of the phycocyanin on the calcium phosphate and
a third step of removing the residue of the cyanobacterium and the adsorbate from the liquid extract.

Furthermore, the extraction method (i) is more preferably the extraction method (ii) below.

### <<Extraction Method (ii) of Phycocyanin>>

The extraction method (ii) has
a first step of obtaining a liquid extract in which phycocyanin in a cyanobacterium is extracted in an aqueous suspension,
a second step of producing calcium phosphate by reacting a calcium salt and a phosphate in the liquid extract and obtaining an adsorbate by adsorbing a contaminant of the phycocyanin on the calcium phosphate,
a third step of removing the residue of the cyanobacterium and the adsorbate from the liquid extract and
a step of adding a chelating agent to the liquid extract before the third step.

Using the extraction method (i) or (ii) of phycocyanin above, phycocyanin with a high quality can be extracted from a cyanobacterium.

In particular, when the extraction method (i) or (ii) above is used on a cyanobacterium of the genus *Spirulina,* phycocyanin having an excellent mixing ratio of C-phycocyanin and allophycocyanin and a high quality can be extracted.

In this regard, in the extraction methods, the mixing ratio of C-phycocyanin and allophycocyanin is preferably adjusted in a desired range by appropriately selecting the extraction conditions.

The kinds of the phycocyanin may be all C-phycocyanin. Alternatively, allophycocyanin may be contained, and a mixture of C-phycocyanin and allophycocyanin may be contained in the composition.

The mixing ratio of C-phycocyanin and allophycocyanin is, for example, preferably, 3 to 9.5:0.5 to 7 as a mass ratio, more preferably 6 to 9.5:0.5 to 4, further preferably 7 to 8:2 to 3.

### <Enzymatically Degraded Spirulina>

The enzymatically degraded spirulina is a kind of active ingredient of the composition according to the embodiment and has an action of improving memory learning function and/or cognitive function and an action of suppressing deterioration of memory learning function and/or cognitive function.

The enzymatically degraded spirulina is an enzymatically degraded product obtained by degrading the proteins, the saccharides and the like of the spirulina described above by causing an enzyme to act.

The enzyme used for the enzymatic degradation is not particularly restricted, and for example, a glucanase, a chitinase, a protease, a pectinase, a lipase, a cellulase, a xylanase, a mannanase, a hemicellulase, a nuclease or the like can be used. Of these, in view of suitable degradation of spirulina, a protease is preferable as the enzyme used for the enzymatic degradation. As the enzyme, a kind alone or a combination of two or more kinds can be used.

Here, the origin of the enzyme used for the enzymatic degradation is not particularly restricted, and for example, *Aspergillus niger, Aspergillus melleus, Aspergillus oryzae, Rhizopus niveus, Bacillus subtilis, Arthrobacter* sp., *Trichoderma viride* or the like can be used.

The protease is not particularly restricted, and a commercial protease preparation can be used. As the protease preparation, for example, Sumizyme LP, Sumizyme FL-G, Sumizyme CP, Sumizyme FP-G, Sumizyme MP (SHINNIHON CHEMICALS Corporation), Bromelain F, Protease P "Amano" 3 SD, Papain W-40, Thermoase PC10F, Thermoase C100, Thermoase C160, Protin SD-NY10 (Protin SD-PC10F) (Amano Enzyme Inc.) or the like can be used.

Regarding the molecular weight distribution of the proteins of the enzymatically degraded spirulina, the proportion of components having a molecular weight of less than 500 is preferably 20 mass% or more, more preferably 30 mass% or more, further preferably 35 mass% or more. When the molecular weight distribution of the proteins of the enzymatically degraded spirulina is the lower limit or more, the absorbability of the enzymatically degraded spirulina into the body is excellent.

The value of the molecular weight distribution can be obtained by analyzing the sample by liquid chromatography using a gel filtration column.

The treatment method of the enzymatic degradation is not particularly restricted, and the treatment can be conducted according to a general method. The reaction conditions of the enzymatic degradation are not particularly restricted, either, and the reaction conditions such as the optimum temperature, the optimum pH and the reaction period may be appropriately adjusted according to the kind, the activity level, the amount and the like of the enzyme. The enzymatically degraded spirulina obtained by the enzymatic degradation treatment can be adjusted to the aimed form and properties through an operation such as centrifugation, filtration, desalination, concentration, drying, solvent extraction, dilution and addition of an additive, according to the need.

An example of the method for preparing the enzymatically degraded spirulina in the composition according to the embodiment is shown below.

An aqueous spirulina solution is heated at 20 to 70°C. After adjusting the aqueous solution to the optimum pH of the enzyme used, an enzyme in an amount of 0.01 mass% or more, preferably 0.1 to 10 mass%, based on the proteins in the spirulina is added, and the mixture is stirred for 1 to 24 hours. After deactivating the enzyme or stopping the enzymatic degradation by heating or cooling after stirring, the mixture is centrifuged. After the centrifugation, the obtained supernatant is freeze-dried, and thus enzymatically degraded spirulina is obtained.

### <Enzymatically Degraded Phycocyanin>

The enzymatically degraded phycocyanin is a kind of active ingredient of the composition according to the embodiment and has an action of improving memory learning function, an action of suppressing deterioration of memory learning function, an action of improving cognitive function and an action of suppressing deterioration of cognitive function.

The enzymatically degraded phycocyanin is an enzymatically degraded product obtained by degrading the phycocyanin described above by causing an enzyme to act.

The enzyme used for the enzymatic degradation is not particularly restricted, and the enzyme explained for the enzymatically degraded spirulina described above can be used. Thus, the explanation is omitted. In this regard, as the enzyme used for the enzymatic degradation, a protease is preferably used as for the enzymatically degraded spirulina described above.

Regarding the molecular weight distribution of the enzymatically degraded phycocyanin, the proportion of components having a molecular weight of less than 500 is preferably 20 mass% or more, more preferably 30 mass% or more, further preferably 35 mass% or more. When the molecular weight distribution of the enzymatically degraded phycocyanin is the lower limit or more, the absorbability of the enzymatically degraded phycocyanin into the body is excellent.

The value of the molecular weight distribution can be obtained by analyzing a sample by liquid chromatography using a gel filtration column.

The protease and the enzymatic degradation treatment method used for the enzymatic degradation are not particularly restricted and are similar to those explained for the enzymatically degraded spirulina described above. Thus, the explanation is omitted.

An example of the method for preparing the enzymatically degraded phycocyanin in the composition according to the embodiment is shown below.

An aqueous phycocyanin solution is heated at 20 to 70°C. After adjusting the aqueous solution to the optimum pH of the enzyme used, an enzyme in an amount of 0.01 mass% or more, preferably 0.1 to 10 mass%, based on the phycocyanin is added, and the mixture is stirred for 1 to 24 hours. After deactivating the enzyme or stopping the enzymatic degradation by heating or cooling after stirring, the mixture is centrifuged. After the centrifugation, the obtained supernatant is freeze-dried, and thus enzymatically degraded phycocyanin is obtained.

### <Composition for Improving Memory Learning Function and/or Cognitive Function or Suppressing Deterioration Thereof>

As shown in the Examples described below, the active ingredients of spirulina, phycocyanin, enzymatically degraded spirulina and enzymatically degraded phycocyanin increased the change rate of spontaneous alternation behavior in a spontaneous alternation behavior test. Of the active ingredients, in particular, enzymatically degraded phycocyanin is particularly preferable because the enzymatically degraded phycocyanin significantly increased the change rate of spontaneous alternation behavior compared to the control group. Moreover, it was observed that the active ingredients relieved facilitation of expression or suppression of expression of genes which were expected to be involved in memory learning function and/or cognitive function. Examples of the genes which are expected to be involved in memory learning function and/or cognitive function include Mpc2 (Brp44, mitochondrial pyruvate carrier 2), Abat (4-aminobutyrate aminotransferase), Cct4 (chaperonin containing Tcp1, subunit 4 (delta), Map9 (Mtap9, microtubule-associated protein 9), Prnp (prion protein), Mgat3 (mannoside acetylglucosaminyltransferase 3), and Vegfd (Figf, vascular endothelial growth factor D).

That is, the active ingredients of spirulina, phycocyanin, enzymatically degraded spirulina and enzymatically degraded phycocyanin have an action of improving memory learning function and/or cognitive function and an action of suppressing deterioration of memory learning function and/or cognitive function.

Accordingly, the composition according to the embodiment can be used as a composition for improving memory learning function and/or cognitive function or suppressing deterioration thereof containing at least one kind selected from the group consisting of spirulina, phycocyanin, enzymatically degraded spirulina and enzymatically degraded phycocyanin as an active ingredient.

### (Food Composition)

The composition according to the embodiment can be provided as a general food as well as a food composition having an aimed action by blending various foods with an active ingredient in an effective amount that can effectively exhibit the action as a food material, in the field of foods. The composition according to the embodiment can be suitably used, for example, as a food composition such as a health food, a functional food, a nutritional supplement, a supplement, a food with a health claim, a food for a specified health use, a food with a nutrient function claim, a food with a function claim, a food for the ill, a food additive, feed and a feed additive. The form of the food composition is not particularly restricted, can be appropriately selected according to the purpose and can be, for example, solid, liquid, gel or the like.

In this regard, the foods with function claims are foods labeled with function claims based on scientific evidence under the business operator's responsibility and are foods whose information on the safety and the evidence of function claims or the like has been submitted to the Secretary-General of the Consumer Affairs Agency before the sales. The food composition according to the embodiment may be labeled, as a food composition for the purpose of an action of improving memory learning function and/or cognitive function and an action of suppressing deterioration of memory learning function and/or cognitive function, with "having a function of maintaining memory as a part of cognitive function (ability of remembering images of words/objects or positional information)", "having a function of improving memory as a part of cognitive function (ability of remembering words or seen objects)", "maintaining memory, attention, judgement and spatial recognition as a part of cognitive function of the middle-aged and elderly people which deteriorate with aging", "having a function suitable for those who are concerned about deterioration of memory with aging (helping maintenance/search/regeneration of memory)", "having a function of supporting memory of information such as numbers/words/figures/circumstances as a part of cognitive function" and the like.

Examples of the food composition include: non-alcoholic beverages such as soft drinks, carbonated drinks, fruit juice-containing drinks, vegetable juice-containing drinks, fruit juice- and vegetable juice-containing drinks, animal milk including cow's milk, soy milk, milk beverages, drink-type yoghurt, drink-type or stick-type jelly, coffee, cocoa, tea drinks, nutritional drinks, energy drinks, sports drinks, mineral water, near water and non-alcoholic beer-tasting beverages; carbohydrate-containing foods and drinks such as rice, noodle, breads and pasta; dairy products such as cheese, hard-type or soft-type yoghurt, fresh cream of animal milk or another fat or oil raw material and ice cream; various kinds of confectioneries such as western confectioneries including cookies, cakes and chocolate, Japanese confectioneries including *manju,* and adzuki-bean jelly, tablet confectioneries (refreshing candies) including fizzing candies, candies, gums, chilled desserts and frozen desserts including jelly and pudding and snacks; alcohols such as whiskey, bourbon, spirits, liqueur, wine, fruit liquor, Japanese rice wine, Chinese wine, *shochu,* beer, non-alcoholic beer with an alcohol content of 1% or less, sparkling wine, other miscellaneous alcoholic drinks and *shochu* with soda water; processed foods using eggs, processed foods of fish and shellfish or livestock meat (including the offal like liver) (including delicacies), processed foods such as soups including miso soup, seasonings such as soybean paste, soy sauce, *Furikake* (dry Japanese seasonings) and other seasonings, liquid foods such as concentrated liquid diets and the like.

Moreover, when the food composition is, for example, a health food, a functional food, a nutritional supplement, a supplement, a food with a health claim, a food for a specified health use, a food with a nutrient function claim, a food with a function claim, a food for the ill, a food additive, feed, a feed additive or the like, the food composition may be tablets (including chewable tablets or the like), capsules, troches, a syrup, jelly, granules, powder or the like.

A kind or two or more kinds of components which can be generally used for a food composition may be freely selected and blended with the food composition according to the embodiment in addition to the active ingredient. For example, all additives which can be generally used in the field of foods, such as various kinds of seasonings, preservatives, emulsifiers, stabilizers, aromas, colorants, antiseptics and pH-adjusting agents, can be contained.

### (Pharmaceutical Composition)

The composition according to the embodiment can be provided as a pharmaceutical composition having an aimed action by blending a pharmaceutically acceptable carrier, an additive or the like together with the active ingredient in an effective amount that can effectively exhibit the action, in the field of pharmaceutical products. The composition according to the embodiment can be used for treating, preventing or improving a disease and a symptom which can be treated, prevented or improved through improvement of memory learning function and/or cognitive function or suppression of deterioration thereof. Examples of the disease and the symptom which can be treated, prevented or improved through improvement of memory learning function and/or cognitive function or suppression of deterioration thereof include memory impairment (a symptom of inability of remembering memory or memorizing a new thing), disorientation (disorientation of time/place/person), and a cognitive dysfunction (deterioration of calculating ability/deterioration of judgement/aphasia/agnosia/apraxia/executive function disorder). More specifically, the composition according to the embodiment can be suitably used, for example, as a pharmaceutical composition for dementia, a pharmaceutical composition for Alzheimer's disease or the like. In this regard, the pharmaceutical composition may be a pharmaceutical product or a quasi-drug.

The form of the pharmaceutical composition is not particularly restricted, can be appropriately selected according to the purpose and can be, for example, solid, liquid, gel or the like.

The pharmaceutical composition can contain an additive or the like such as a carrier, a binder, a stabilizing agent, an excipient, a diluent, a pH buffer, a disintegrant, a solubilizer, a solubilizing agent and an isotonic agent which are pharmaceutically acceptable and generally used. The pharmaceutical composition may be for oral use or parenteral use but is more preferably for oral use. For oral use, a generally used form of administration, for example, a dosage form of tablets, powder, granules, capsules, a syrup, a suspension or the like, can be used. For parenteral use, a generally used form of administration, for example, injection (subcutaneous injection, intravenous injection, intramuscular injection or the like) in the dosage form of a solution, an emulsion, a suspension or the like, nasal administration in the dosage form of a spray preparation or the like, can be used.

Examples in which the form of the food composition and/or the pharmaceutical composition is tablets are shown below.

When the form of the food composition and/or the pharmaceutical composition is, for example, tablets, the compositions can be prepared according to a general method by appropriately combining the active ingredient with an additive such as an excipient, a binder, a disintegrant, a lubricant, a preservative, an antioxidant, an isotonic agent, a buffer, a coating agent, a corrigent, a solubilizing agent, a base, a dispersing agent, a stabilizing agent and a colorant.

The excipient may be starch or a derivative thereof (dextrin, carboxymethyl starch or the like), cellulose or a derivative thereof (methylcellulose, hydroxypropyl methylcellulose or the like), a saccharide (lactose, sucrose, glucose, trehalose or the like), citric acid or a salt thereof, malic acid or a salt thereof, ethylenediaminetetraacetic acid or a salt thereof.

The binder may be starch or a derivative thereof (gelatinized starch, dextrin or the like), cellulose or a derivative thereof (ethyl cellulose, sodium carboxymethylcellulose, hydroxypropyl methylcellulose or the like), gum arabic, traganth, gelatin, a saccharide (glucose, sucrose or the like), ethanol or the like.

The disintegrant may be starch or a derivative thereof (carboxymethyl starch, hydroxypropyl starch or the like), cellulose or a derivative thereof (sodium carboxymethylcellulose, crystalline cellulose, hydroxypropyl methylcellulose or the like), a carbonate (calcium carbonate, calcium hydrogen carbonate or the like), traganth, gelatin, agar or the like.

The lubricant may be stearic acid, calcium stearate, magnesium stearate, talc, titanium oxide, calcium hydrogen phosphate, dried aluminum hydroxide gel, a sucrose fatty acid ester, edible oil or fat or the like.

The preservative may be a paraoxybenzoate ester, a sulfite (sodium sulfite, sodium pyrosulfite or the like), a phosphate (sodium phosphate, calcium polyphosphate, sodium polyphosphate, sodium metaphosphate or the like), dehydroacetic acid, sodium dehydroacetate, glycerol sorbate, a saccharide or the like.

The antioxidant may be a sulfite (sodium sulfite, sodium hydrogen sulfite or the like), erythorbic acid, L-ascorbic acid, cysteine, thioglycerol, butylated hydroxyanisole, dibutyl hydroxy toluene, propyl gallate, ascorbyl palmitate, dl-α-tocopherol or the like.

The isotonic agent may be sodium chloride, sodium nitrate, potassium nitrate, dextrin, glycerin, glucose or the like.

The buffer may be sodium carbonate, hydrochloric acid, boric acid, a phosphate (sodium hydrogen phosphate or the like) or the like.

The coating agent may be a cellulose derivative (hydroxypropyl cellulose, cellulose acetate phthalate, hydroxypropyl methylcellulose phthalate or the like), shellac, polyvinylpyrrolidone, a polyvinyl pyridine (poly-2-vinylpyridine, poly-2-vinyl-5-ethylpyridine or the like), polyvinyl acetyl diethyl amino acetate, polyvinyl alcohol phthalate, methacrylate/methacrylic acid copolymer or the like.

The corrigent may be a saccharide (glucose, sucrose, lactose or the like), sodium saccharin, a sugar alcohol or the like.

The solubilizing agent may be ethylene diamine, nicotinamide, sodium saccharin, citric acid, a citrate, sodium benzoate, polyvinylpyrrolidone, a polysorbate, a sorbitan fatty acid ester, glycerin, polyprene glycol, benzyl alcohol or the like.

The base may be fat (lard or the like), plant oil (olive oil, sesame oil or the like), animal oil, lanolin acid, Vaseline, paraffin, resin, bentonite, glycerin, glycol oil or the like.

The dispersing agent may be gum arabic, traganth, a cellulose derivative (methylcellulose or the like), sodium alginate, a polysorbate, a sorbitan fatty acid ester or the like.

The stabilizing agent may be a sulfite (sodium hydrogen sulfite or the like), nitrogen, carbon dioxide or the like.

The total active ingredient content of the food composition and/or the pharmaceutical composition according to the embodiment (the total amount of the amounts of all the active ingredients contained in the composition) differs with the conditions such as the kind, the components and the form of the food or the pharmaceutical product, is not particularly restricted in the range in which the effects of the invention are obtained and can be appropriately selected.

Moreover, in particular, when the form of the food composition and/or the pharmaceutical composition according to the embodiment is tablets, the total active ingredient content of the tablets is not particularly restricted in the range in which the effects of the invention are obtained but is preferably, in the total mass of the food composition and/or the pharmaceutical composition in terms of the dry weight of the active ingredient, 20 mass% or more, more preferably 50 mass% or more. Moreover, the total content may be 100 mass% or less and is preferably 99 mass% or less.

In the invention, the total intake of the active ingredient is not particularly limited and is appropriately selected depending on the kind, the components and the like of the food or the pharmaceutical product, but, for example, the intake per adult per day in terms of the dry weight of the active ingredient is preferably 0.01 g or more, more preferably 0.03 g or more and is preferably 10 g or less, more preferably 4 g or less.

### EXAMPLES

Although the invention is further explained in detail referring to Examples below, the scope of the invention should not be limited to the Examples.

### [Test Example 1]

Using amyloid β₂₅₋₃₅-induced memory impairment model mice, the influence of spirulina, phycocyanin, enzymatically degraded spirulina and enzymatically degraded phycocyanin on memory learning function and/or cognitive function was examined.

### <Test Substances>

As the test substances, spirulina, phycocyanin, enzymatically degraded spirulina and enzymatically degraded phycocyanin were used.

### <Spirulina>

*Spirulina platensis* was proliferated in an outdoor culture pond under basic conditions (pH 11). Next, 50 g of algal powder obtained by spray-drying the proliferated *Spirulina platensis* was suspended in 500 mL of distilled water in an autoclave, and extraction was conducted for an hour at an extraction temperature of 120°C by adjusting the pressure.

The pH of the liquid extract was adjusted to 4.0 with citric acid. The algal residue and the protein (insoluble fraction) were removed therefrom through centrifugation, and a spirulina extract which was a spirulina hot-water extraction liquid was thus obtained. The obtained spirulina extract was spray-dried and then crushed, and thus spirulina powder was obtained.

### <<Phycocyanin>>

To 1300 L of 1% calcium chloride (anhydrous) solution, 65 kg of a dried spirulina alga produced in an outdoor culture tank (a spray-dried product) was added, and a uniform suspension was obtained by stirring for 15 minutes. Then, phycocyanin in the cyanobacterium was extracted into the solution at 20°C for 15 hours under static conditions, and thus a liquid extract was obtained.

To the liquid extract, 32 kg of sodium dihydrogen phosphate was added, and after stirring for 0.5 hours, the reaction was conducted at 20°C under static conditions for 2.5 hours. Thus, calcium phosphate was produced, and an adsorbate was obtained by adsorbing contaminants of phycocyanin on the calcium phosphate. Then, the liquid extract was introduced to a centrifuge and centrifuged at a gravitational acceleration of 10,000 G for 15 minutes to remove the residue of the cyanobacterium and the adsorbate from the liquid extract. Small molecule components and salts were removed from the obtained liquid extract of phycocyanin through ultrafiltration using a separation membrane having a molecular cutoff of 10,000, and then, after adding trehalose and trisodium citrate, the mixture was mixed and spray-dried. Thus, 15 kg of a dried phycocyanin pigment product was obtained. This was used as the phycocyanin. Here, the phycocyanin content of 100 mass% of the phycocyanin pigment powder was about 30 mass% (C-phycocyanin was about 22 mass%, and allophycocyanin was about 8 mass%.

### <<Enzymatically Degraded Spirulina>>

After dissolving 100 g of the spirulina in 1400 mL of distilled water, the solution was heated to 50 to 52°C. Next, a 1N sodium hydroxide solution was added to adjust the pH of the aqueous solution to 7.0. After adding 2 mass% of Protin (SD-NY10, manufactured by Amano Enzyme Inc.), based on the proteins in the spirulina, to the aqueous solution, the mixture was stirred at 50 to 52°C for six hours, and the spirulina was enzymatically degraded. The solution after the reaction was cooled to room temperature, then introduced to a centrifuge and centrifuged at a gravitational acceleration of 10,000 G for 15 minutes. The supernatant was taken and then freeze-dried, and thus 62 g of enzymatically degraded spirulina, which was a protease-degraded product of spirulina, was obtained.

### «Enzymatically Degraded Phycocyanin»

After dissolving 17 g of the phycocyanin in 1400 mL of distilled water, the solution was heated to 50 to 52°C. Next, 1N hydrochloric acid was added to adjust the pH of the aqueous solution to 7.0. After adding 2 mass% of Protin (SD-NY10, manufactured by Amano Enzyme Inc.), based on the phycocyanin, to the aqueous solution, the mixture was stirred at 50 to 52°C for six hours, and the phycocyanin was enzymatically degraded. The solution after the reaction was cooled to room temperature, then introduced to a centrifuge and centrifuged at a gravitational acceleration of 10,000 G for 15 minutes. The supernatant was taken and then freeze-dried, and thus 16 g of enzymatically degraded phycocyanin, which was a protease-degraded product of phycocyanin, was obtained.

### <Production of Amyloid β₂₅₋₃₅-Induced Memory Impairment Model Mice>

Slc:ddY male mice (Japan SLC, Inc.) were used. Amyloid β₂₅₋₃₅-induced memory impairment model mice were produced as described below.

Amyloid β-Protein₂₅₋₃₅ (amyloid P₂₅₋₃₅, 0.51 µmol, manufactured by Peptide Institute, Inc.) in an amount of 0.54 mg was dissolved in 250 µL of water for injection added thereto. The solution which was incubated at 37°C for four days was used as the amyloid β₂₅₋₃₅ administration solution. Seven days before conducting a spontaneous alternation behavior test, the amyloid β₂₅₋₃₅ administration solution was intraventricularly administered (administered dose of 10 nmol/mouse), and thus amyloid β₂₅₋₃₅-induced memory impairment model mice were produced.

In this regard, it is known that, in amyloid β₂₅₋₃₅-induced memory impairment model mice, inflammatory response caused by aggregation/deposition of amyloid β₂₅₋₃₅ and cognitive dysfunction with cell death can be imitated.

### <Test System>

The experiment groups, the test substances, the intraventricularly administered substances and the case numbers are shown in Table 1 below. Regarding the number of administration and the administration period of the test substances, the test substances were orally administered for 22 days from 22 days before conducting the spontaneous alternation behavior test at a frequency of once a day (administered volume of test substance: 10 mL/kg). In this regard, group A1 is also called the Sham group (the normal mouse group without the intraventricular administration of amyloid β₂₅₋₃₅), and group A2 is also called the medium group.

**[Table 1]**

| Experiment Group | Test Substance | | Intraventricularly Administered Substance | | Case Number |
|---|---|---|---|---|---|
| | Test Substance Name | Administered Dose [mg/kg] | Intraventricularly Administered Substance Name | Administered Dose [nmol/mouse] | |
| A1 (Sham Group) | Water for Injection (medium) | - | Water for Injection (medium) | - | 4 |
| A2 (Medium Group) | Water for Injection (medium) | - | Amyloid β₂₅₋₃₅ | 10 | 6 |
| A3 | Spirulina | 750 | Amyloid β₂₅₋₃₅ | 10 | 6 |
| A4 | Enzymatically Degraded Spirulina | 750 | Amyloid β₂₅₋₃₅ | 10 | 6 |
| A5 | Enzymatically Degraded Phycocyanin | 750 | Amyloid β₂₅₋₃₅ | 10 | 6 |
| A6 | Phycocyanin | 120 | Amyloid β₂₅₋₃₅ | 10 | 6 |

### <Spontaneous Alternation Behavior Test>

As the spontaneous alternation behavior test, a Y-maze test for evaluating spontaneous alternation behavior was conducted. Mice have nature to select a different route from the route which has been just selected. Accordingly, when a mouse is placed in a Y maze having three arms having equal widths, lengths and the like, the mouse generally enters a different arm from the arm to which the mouse has just entered. A Y-maze test, which is a spontaneous alternation behavior test, is a test using the nature of mice to evaluate short-term memory.

### <<Device of Spontaneous Alternation Behavior Test>>

As the experiment device, a Y maze in which three arms having an arm length of 40 cm, a wall height of 12 cm, a floor width of 3 cm and an upper width of 10 cm were connected at an angle of 120 degrees was used.

### <<Measurement Method of Change Rate of Spontaneous Alternation Behavior>>

A mouse was placed at the end of one of the arms of the Y maze 60 minutes after the final administration of a test substance or the medium and was allowed to explore freely in the maze for eight minutes, and the positions of the arms in which the mouse entered were recorded in the selected order. The number of the entries of the mouse to the arms during the measurement period was counted and regarded as the total entry number. Of these, the combinations in which different three arms were selected in a row (for example, the case of the arms of entries of ABCBACACB is considered 4 including the overlap) were examined, and the number was regarded as the spontaneous alternation behavior number. The spontaneous alternation behavior number was divided by the number obtained by subtracting 2 from the total entry number. The value obtained by multiplying this by 100 was regarded as the change rate of spontaneous alternation behavior, and this was regarded as the indicator of spontaneous alternation behavior. As the change rate of spontaneous alternation behavior is higher, the short-term memory is maintained. The obtained change rates of spontaneous alternation behavior of animals with a total entry number of 8 or less were not considered to reflect the accurate spontaneous alternation behavior, and the scores of the animals were excluded from the test.

### <DNA Microarray>

The mice were decapitated after the completion of the measurement of spontaneous alternation behavior, and the whole brains were taken out. The hippocampi were taken from the extracted brains, and after measuring the wet weights (mg), the hippocampi were treated with RNAlater and cryopreserved (-70°C or lower). In this regard, it is known that hippocampus is involved in memory of cognitive function and is one of the parts in which lesion is observed in Alzheimer's disease.

RNA was extracted from the hippocampi of groups A1, A2 and A5 and group A6 and was used for DNA microarrays. The DNA microarrays were obtained as described below.

Pools of the RNA samples were obtained for the respective groups, and cDNA synthesis and synthesis and purification of Cy3-labeled cRNA were conducted using Low Input Quick Amp Labeling Kit (Agilent). The concentrations of the obtained labeled cRNA and the incorporation of Cy3 were calculated from the absorbances at 260 nm, 280 nm, 550 nm and 320 nm, and it was found that the standard value (Cy3-CTP incorporation > 6 pmol/µg) was satisfied. Next, using Gene Expression Hybridization Kit (Agilent), the labeled cRNA was fragmented, applied to Whole Mouse Genome Microarray Ver2.0 (Agilent) and hybridized at 65°C for 17 hours. Next, using Gene Expression Wash Buffers 1 and 2 (Agilent), the array slides were washed. The array images which were scanned with a microarray scanner were quantified using an array analysis software GenePix Pro (Molecular Devices). The fluorescence intensity values were normalized, and the proportions of the groups based on group A1 were calculated.

### <Results of Spontaneous Alternation Behavior Test>

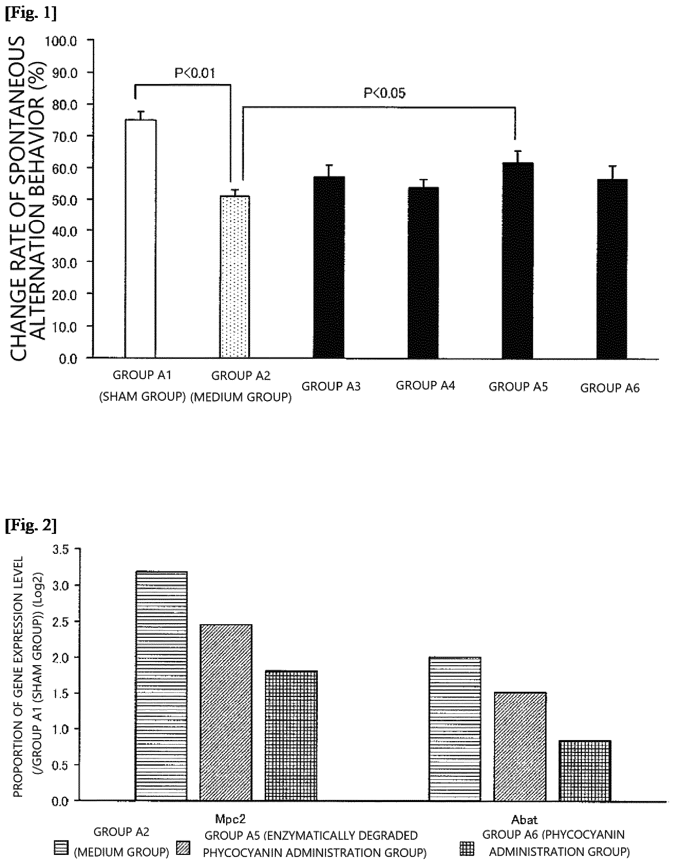

The results of the spontaneous alternation behavior test are shown in Table 2 and Fig. 1. Table 2 is a table showing the results of the total entry numbers, the spontaneous alternation behavior numbers and the change rates of spontaneous alternation behavior. Fig. 1 is a figure showing the results of the change rates of spontaneous alternation behavior.

**[Table 2]**

| Experiment Group | Case Number | Total Entry Number | | Spontaneous Alternation Behavior Number | | Change Rate of Spontaneous Alternation Behavior [%] | |
|---|---|---|---|---|---|---|---|
| | | Average Value | Standard Error | Average Value | Standard Error | Average Value | Standard Error |
| A1 (Sham Group) | 4 | 26.0 | 5.7 | 17.8 | 4.0 | 75.1 | 2.5 |
| A2 (Medium Group) | 6 | 32.0 | 2.5 | 15.3 | 1.5 | 51.0** | 2.1 |
| A3 | 6 | 30.5 | 2.5 | 16.5 | 2.0 | 57.2 | 3.7 |
| A4 | 6 | 31.0 | 3.4 | 16.0 | 2.5 | 53.9 | 2.5 |
| A5 | 5* | 33.4 | 5.7 | 19.2 | 3.3 | 61.8# | 3.7 |
| A6 | 6 | 24.5 | 3.2 | 13.0 | 2.4 | 56.6 | 4.5 |

| | | | | | | | |
|---|---|---|---|---|---|---|---|
| **P<0.01; vs group A1 + group A2 (Student-t test). #P<0.05; vs group A2 + groups A3-A6 (Student-t test). *N=5 because one mouse died on the next day of the amyloid β administration. | | | | | | | |

As shown in Table 2 and Fig. 1, a significant decrease was observed in the change rate of spontaneous alternation behavior of the medium group (with the intraventricular administration of amyloid β₂₅₋₃₅), which is group A2, through the intraventricular administration of amyloid β₂₅₋₃₅ compared to the Sham group (without the intraventricular administration of amyloid β₂₅₋₃₅), which is group A1.

As shown in Table 2 and Fig. 1, the change rates of spontaneous alternation behavior of all the groups with the test substance administration (groups A3 to A6) were higher than that of the medium group (without the intraventricular administration of amyloid β₂₅₋₃₅), which is group A1. In particular, the change rate of spontaneous alternation behavior of group A5, to which the enzymatically degraded phycocyanin was administered, was significantly higher than that of the medium group (without the intraventricular administration of amyloid β₂₅₋₃₅), which is group A1.

Accordingly, it was found that spirulina, phycocyanin, enzymatically degraded spirulina and enzymatically degraded phycocyanin have an effect of enhancing short-term memory and have an action of improving memory learning function and/or cognitive function and an action of suppressing deterioration of memory learning function and/or cognitive function.

Of the active ingredients, in particular, the change rate of spontaneous alternation behavior of the enzymatically degraded phycocyanin was significantly higher than that of the medium group, and thus it was found that the action of improving memory learning function and/or cognitive function and the action of suppressing deterioration of memory learning function and/or cognitive function thereof were particularly excellent.

### <Results of DNA Microarrays>

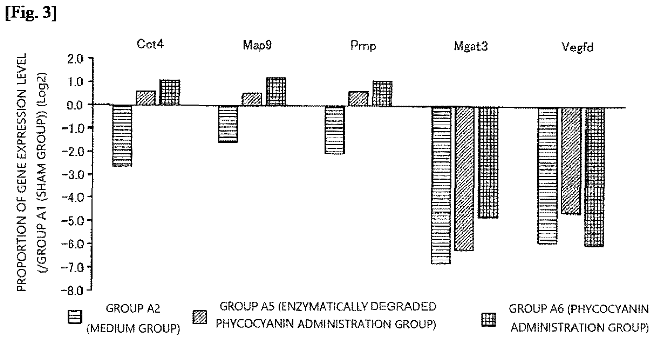

The results of the DNA microarrays are shown in Fig. 2 and Fig. 3.

Fig. 2 is a figure showing the proportions of the gene expression levels of genes in which the facilitation of gene expression by amyloid β₂₅₋₃₅ was relieved based on the Sham group.

As shown in Fig. 2, it was found that the phycocyanin and the enzymatically degraded phycocyanin relieved the abnormal facilitation of expression of the genes by the intraventricular administration of amyloid β₂₅₋₃₅.

Mpc2 and Abat genes shown in Fig. 2 have been indicated to be related to Alzheimer's disease (Rossi, A., Rigotto, G., Valente, G., Giorgio, V, Basso, E., Filadi, R., Pizzo, P. (2020). Defective Mitochondrial Pyruvate Flux Affects Cell Bioenergetics in Alzheimer's Disease-Related Models. Cell reports, 30, 2332-2348 and Ciminelli, B. M., Menduti, G., Benussi, L., Ghidoni, R., Binetti, G., Squitti, R., et al. (2020). Polymorphic Genetic Markers of the GABA Catabolism Pathway in Alzheimer's Disease. Journal of Alzheimer's disease: JAD, 77, 301-31). Mpc2 is required for ATP production as a pyruvate carrier in the mitochondrial TCA cycle and is known to play an important role in maintenance of mitochondrial functions and thus maintenance of cell functions. Abat is related also to the salvage function in mitochondria (nucleic acid recycling) but is a GABA- (γ-aminobutyric acid, an inhibitory neurotransmitter) degrading enzyme and is essential for regulating the brain GABA amount. That the phycocyanin and the enzymatically degraded phycocyanin relieved the abnormal expression of the genes suggests that the substances may return the mitochondrial dysfunction due to intraventricular amyloid β₂₅₋₃₅ to the direction of normalization, contributes to homeostasis of the GABA amount and relieve the pathological conditions of Alzheimer's disease. Accordingly, the phycocyanin and the enzymatically degraded phycocyanin relieved the facilitation of expression of Mpc2 and Abat and thus were found to have an action of improving memory learning function and/or cognitive function and an action of suppressing deterioration of memory learning function and/or cognitive function. Moreover, from the results, it was speculated that spirulina and enzymatically degraded spirulina also similarly exhibit an action of improving memory learning function and/or cognitive function and an action of suppressing deterioration of memory learning function and/or cognitive function.

Fig. 3 is a figure showing the proportions of the gene expression levels of genes in which the suppression of gene expression by amyloid β₂₅₋₃₅ was relieved based on the Sham group.

As shown in Fig. 3, it was found that the phycocyanin and the enzymatically degraded phycocyanin relieved the abnormal suppression of expression of the genes by the intraventricular administration of amyloid β₂₅₋₃₅.

Cct4 and Map9 shown in Fig. 3 are known as genes required for maintaining the microtubular structure. As shown in Fig. 3, the suppression of gene expression of Cct4 and Map9 was relieved, or there was rather a tendency towards facilitation of expression. Alzheimer's disease is characterized by breakdown of the microtubular structure, and it is thus believed that phycocyanin and enzymatically degraded phycocyanin may contribute to maintenance of the microtubular structure and relieve the pathological conditions.

Moreover, as shown in Fig. 3, the suppression of gene expression also of Prnp, which encodes prion protein, was relieved, or there was rather a tendency towards facilitation of expression. Abnormal prion causes a prion disease such as Creutzfeldt-Jakob disease and is related also to Alzheimer's disease. On the other hand, normal prion is related to autophagy function and is required for protecting nerve cells.

Furthermore, the suppression of expression of Mgat3, which attracts attention as a target gene for treating Alzheimer's disease, shown in Fig. 3 was relieved by the phycocyanin. The expression of Mgat3 is facilitated in patients with Alzheimer's disease, and the modification of BACE1 by bisecting GlcNAc of the specific sugar protein biologically synthesized by Mgat3 is known to be largely involved in abnormal production of amyloid β by BACE1.

Moreover, the suppression of expression of Vegfd shown in Fig. 3 was relieved by the enzymatically degraded phycocyanin. Vegfd is required to recover the form/functions of dendrites in neuronopathy, and it has been reported that nasal administration of a Vegfd analog improves brain damage in ischemic stroke model mice (Mauceri, D., Buchthal, B., Hemstedt, T. J., Weiss, U., Klein, C. D., Bading, H. (2020). Nasally delivered VEGFD mimetics mitigate stroke-induced dendrite loss and brain damage. Proceedings of the National Academy of Sciences of the United States of America, 117, 8616-8623). It is believed that phycocyanin and enzymatically degraded phycocyanin return the expression of Vegfd gene to normal state and thus may have an effect of protecting nerve cells including maintenance of neuronal dendrites.

The phycocyanin and the enzymatically degraded phycocyanin were found to relieve the suppression of expression of Cct4, Map9, Prnp, Mgat3 and Vegfd. Accordingly, it was found that phycocyanin and enzymatically degraded phycocyanin have an action of improving memory learning function and/or cognitive function and an action of suppressing deterioration of memory learning function and/or cognitive function. Moreover, from the results, it was speculated that spirulina and enzymatically degraded spirulina also similarly exhibit an action of improving memory learning function and/or cognitive function and an action of suppressing deterioration of memory learning function and/or cognitive function.

From the results of the spontaneous alternation behavior test and the results of the DNA microarrays above, it was found that a composition containing at least one kind selected from the group consisting of spirulina, phycocyanin, enzymatically degraded spirulina and enzymatically degraded phycocyanin as an active ingredient is a composition exhibiting an action of improving memory learning function and/or cognitive function and an action of suppressing deterioration of memory learning function and/or cognitive function.

## Claims

1. A composition for improving memory learning function and/or cognitive function or suppressing deterioration thereof, comprising at least one kind selected from the group consisting of spirulina, phycocyanin, enzymatically degraded spirulina and enzymatically degraded phycocyanin as an active ingredient.

2. The composition according to claim 1, wherein the enzyme in the enzymatically degraded spirulina and the enzymatically degraded phycocyanin is a protease.

3. The composition according to claim 1 or 2, wherein the phycocyanin is phycocyanin derived from a cyanobacterium.

4. The composition according to claim 3, wherein the cyanobacterium is a cyanobacterium of the genus *Spirulina.*

5. The composition according to any one of claims 1 to 4, wherein the active ingredient is the phycocyanin or the enzymatically degraded phycocyanin.

6. The composition according to any one of claims 1 to 5 which is a food composition.

7. The composition according to claim 6 which is a health food, a functional food, a nutritional supplement, a supplement, a food with a health claim, a food for a specified health use, a food with a nutrient function claim, a food with a function claim or a food for the ill.

8. The composition according to any one of claims 1 to 5 which is a pharmaceutical composition.

9. The composition according to claim 8 which is a pharmaceutical composition for dementia or Alzheimer's disease.
